# EUROPEAN PATENT SPECIFICATION

(11) **EP 2 399 134 B1**
(45) Date of publication and mention of the grant of the patent: **02.04.2014**
(21) Application number: 10708952.6
(22) Date of filing: 17.02.2010
(51) Int. Cl.: G01N 33/68

(54) **GDF15 AS MOLECULAR TOOL TO MONITOR AND ENHANCE PHENOTYPIC STABILITY OF ARTICULAR CHONDROCYTES.**
GDF15 ALS MOLEKULARES WERKZEUG ZUR ÜBERWACHUNG UND VERBESSERUNG DER PHÄNOTYPISCHEN STABILITÄT ARTIKULÄRER CHONDROZYTEN
GDF15 UTILISÉ COMME OUTIL MOLÉCULAIRE POUR SURVEILLER ET AMÉLIORER LA STABILITÉ PHÉNOTYPIQUE DE CHONDROCYTES ARTICULAIRES

(30) Priority: 20.02.2009 GB 0902793
(43) Date of publication of application: 28.12.2011
(73) Proprietor: Universiteit Gent, 9000 Gent (BE)
(72) Inventor: LAMBRECHT, Stijn, 9090 Melle (BE); DEFORCE, Dieter, 8520 Kuurne (BE); ELEWAUT, Dirk, 9070 Heusden (BE); VERBRUGGEN, August, 9810 Nazareth (BE)
(74) Representative: Laenen, Bart Roger Albert
(86) International application number: PCT/EP2010/051995
(87) International publication number: WO 2010/094711

(56) References cited:
- EP-A2- 1 498 146
- WO-A1-99/06445
- WO-A2-2008/061804
- ILIOPOULOS DIMITRIOS ET AL.: "Integrative MicroRNA amd Proteomic Approaches Identify Novel Osteoarthritis and Their Collaborative Metabolic and Inflammatory Networks" PLOS ONE, PUBLIC LIBRARY OF SCIENCE, SAN FRANCISCO, CA, US, vol. 3, no. 11, 1 November 2008 (2008-11-01), XP002589646 ISSN: 1932-6203 cited in the application
- HOPWOOD BLAIR ET AL: "Microarray gene expression profiling of osteoarthritic bone suggests altered bone remodelling, WNT and transforming growth factor-Î/bone morphogenic protein signalling" ARTHRITIS RESEARCH AND THERAPY, BIOMED CENTRAL, LONDON, GB, vol. 9, no. 5, 27 September 2007 (2007-09-27), page R100, XP021041138 ISSN: 1478-6354 cited in the application
- NOORALI S ET AL: "Dynamics of expression of growth differentiation factor 15 in normal and PIN development in the mouse" DIFFERENTIATION, SPRINGER VERLAG, DE LNKD- DOI:10.1111/J.1432-0436.2006.00142.X, vol. 75, no. 4, 1 April 2007 (2007-04-01), pages 325-336, XP026765261 ISSN: 0301-4681 [retrieved on 2007-04-01] cited in the application
- HSIAO E C ET AL: "Characterization of growth-differentiation factor 15, a transforming growth factor beta superfamily member induced following liver injury" MOLECULAR AND CELLULAR BIOLOGY, AMERICAN SOCIETY FOR MICROBIOLOGY, WASHINGTON, US LNKD- DOI:10.1128/MCB.20.10.3742-3751.2000, vol. 20, no. 10, 1 May 2000 (2000-05-01), pages 3742-3751, XP002525556 ISSN: 0270-7306 cited in the application
- DELL'ACCIO F ET AL: "Molecular markers predictive of the capacity of expanded human articular chondrocytes to form stable cartilage in vivo" ARTHRITIS & RHEUMATISM, JOHN WILEY & SONS, INC, US LNKD- DOI:10.1002/1529-0131(200107)44:7<1608::AI D-ART284>3.0.CO;2-T, vol. 44, no. 7, 1 July 2001 (2001-07-01), pages 1608-1619, XP002265720 ISSN: 0004-3591

## Description

The invention relates generally to the field of tissue engineering. The present invention relates to GDF15 as a molecular marker in *in vitro* assays determining phenotypic stability of articular chondrocytes and predicting the outcome of chondrocyte transplantation.

The invention further provides methods and compositions related to the generation of a population of cells suitable for the repair of cartilage, in particular in the repair of cartilage degeneration associated with osteoarthritis.

### BACKGROUND OF THE INVENTION

The transforming growth factor-β (TGF-β) superfamily consists of an increasing number of molecules that regulate a variety of cellular processes such as growth, differentiation and oncogenesis. Members of the TGF-β superfamily have been classified into major family groupings which include TGF-β, morphogenic proteins (MP), bone morphogenic proteins (BMP), osteogenic proteins (OP), growth and differentiation factors (GDF), inhibins/activins, mullerian inhibitory substances (MIS) and glial derived neurotrophic factors (GDNF). TGF-β was first characterized for its effects on cell proliferation. It both stimulated the anchorage-independent growth of rat kidney fibroblasts and inhibited the growth of monkey kidney cells. TGF-β family members have been shown to have many diverse biological effects, e.g. they regulate bone formation, induce rat muscle cells to produce cartilage-specific macromolecules, inhibit the growth of early hematopoietic progenitor cells, T cells, B cells, mouse keratinocytes, and several human cancer cell lines. TGF-β family members increase the synthesis and secretion of collagen and fibronectin, accelerate healing of incisional wounds, suppress casein synthesis in mouse mammary explants, inhibit DNA synthesis in rat liver epithelial cells, stimulate the production of bFGF binding proteoglycans, modulate phosphorylation of the epidermal growth factor ("EGF") receptor and proliferation of epidermoid carcinoma cells and can lead to apoptosis in uterine epithelial cells, cultured hepatocytes and regressing liver. TGF-βs can mediate cardio-protection against reperfusion injury by inhibiting neutrophil adherence to endothelium and protect against experimental autoimmune diseases in mice. On the whole, proteins of the TGF-β family are multifunctional, active growth factors and also have related biological activities such as chemotactic attraction of cells, promotion of cell differentiation and tissue-inducing capabilities. Differences in their structure and in their affmity for receptors lead to considerable variations in their exact biological function.

Proteins of the TGF-β family are synthesized as large, inactive precursor (pro-form) proteins, which are proteolytically processed at a dibasic site (RXXR) to generate the mature, active form of the protein.

Growth differentiation factor 15 is a distant member of the TGF-β family. The expression of the mature form of growth differentiation factor 15 (GDF15) protein is associated with early prostate carcinogenesis. GDF 15 has been described in the literature as macrophage inhibitory cytokine-1 (MIC-1), placental bone morphogenic protein (PLAB), placental transforming growth factor- β (PTGF- β), prostate derived factor (PDF), and non-steroidal anti-inflammatory activated gene-1 (NAG-1) reflecting the different functions that has been implied for this protein.

WO2001081928 published on 1 November 2001 discloses diagnostic assays and methods of treatment involving GDF15, and WO1999006445 published on 11 February 1999 discloses the GDF15 polynucleotide sequence and amino acid sequence.

In earlier studies on GDF15 expression in human normal and prostate cancer cells, it was found that the level of GDF15 transcript does not necessarily correlate well with that of the GDF15 protein (Noorali et al. Differentiation (2007) 75: 325-336). The reasons for these discrepancies could be due to differences in post-transcriptional modifications or processing of RNA, that influences its stability or translation, or post-translational modifications of the protein affecting protein maturation, accumulation or degradation.

As indicated above, GDF-15 may have applications in the treatment of immunologic disorders. In particular, GDF-15 may be used as an anti-inflammatory agent or as a treatment for disorders related to abnormal proliferation or function of lymphocytes.

Cartilage is a tissue composed by a cellular component, chondrocytes, and by an extra-cellular matrix typically rich in collagen type II and highly sulphated high molecular weight proteoglycan aggregates. The abundance of type II collagen, link protein, and proteoglycan aggrecan, along with the presence of minor collagens such as type IX and type XI collagen are hallmarks of cartilage tissue. Articular cartilage forms a specialized, smooth connective tissue that is weight bearing and that serves as a gliding surface allowing a lithe movement of the joints.

In post-natal mammals, cartilage contributes to the structure of several organs and systems like the articular surface of diarthrodial joints and other joint-associated structures (such as menisci), the ear, the nose, the larynx, the trachea, the bronchi, structures of the heart valves, part of the costae, synchondroses, entheses etc. In some of the mentioned locations (e.g. entheses, the annulus fibrosus of the intervertebral disks, in the menisci, insertion of ligaments etc.) for the abundance of collagens (mostly type I collagen) and the peculiar distribution of the fibrous bundles it is called *fibrocartilage.* In other locations (e.g. the pinna of the ear, epiglottis etc.) it is particularly rich of elastin and it is called *elastic cartilage.* In all the other structures, for its semitransparent, clear aspect it is called *hyaline cartilage.*

During embryogenesis cartilage has a role in the development of long bones. Mesenchymal cells aggregate and differentiate to form cartilage anlagen, which provide the mold of the future long bones. These cartilage templates in development evolve, undergo endochondral bone formation through a cascade of events including chondrocyte hypertrophy, vascular invasion, mineralization, and are eventually replaced by bone except for a thin layer at the extremities of the bone elements that will differentiate into the articular surface of diarthrodial joints. In these locations cartilage tissue remains hyaline for all the life-span of the individual. With ageing, articular cartilage is well known to undergo a process of senescence, affecting its mechanical properties and its intrinsic resilience.

Joint surface defects can be the result of various aetiologies such as inflammatory processes, neoplasias, post-traumatic and degenerative events etc. Whatever the cause, the mechanisms of repair and of subsequent evolution are largely common.

Osteochondral (or full-thickness) articular surface defects include damage to the articular cartilage, the underlying subchondral bone tissue, and the calcified layer of cartilage located between the articular cartilage and the subchondral bone. They typically arise during severe trauma of the joint or during the late stages of degenerative joint diseases, e.g. during osteoarthritis (OA). These lesions disrupt the congruence between the joint surfaces and therefore can lead to OA, which can be painful and severely limit the joint function. Osteochondral defects can rely on an extrinsic mechanism for repair. Extrinsic healing uses mesenchymal elements from subchondral bone to participate in the formation of new connective tissue. The repair tissue , however, often consists of fibrocartilage or fibrous tissue. This scar tissue does not share the same biomechanical properties as hyaline cartilage and eventually degenerates with the development of osteoarthritis.

Superficial or partial-thickness injuries of the articular cartilage that do not penetrate the subchondral bone can only rely on an intrinsic mechanism for repair. Chondrocytes adjacent to the injured surfaces proliferate and increase the deposition of extracellular matrix synthesis. Despite these attempts at repair, there is no appreciable increase in the bulk of cartilage matrix and the repair process is rarely effective in healing the defects. Although initially sometimes painless, partial-thickness defects often degenerate into osteoarthritis of the involved joint.

Osteoarthritis (OA) is a complex, multifactorial, age-dependent degenerative disease of the synovial joints. It affects females at a higher rate than males, particularly after the menopause. As indicated above OA is characterized by changes to all the components of the joint, with degeneration and loss of articular cartilage and changes to the subchondral bone being constant factors in disease progression. Along with the breakdown of the cartilage and joint space narrowing, there is thickening and sclerosis of the subchondral bone, development of cysts and bony outgrowth at the margins of the joint. Despite an increase in bone volume fraction, the subchondral bone is mechanically weaker in OA because of hypomineralization, increased collagen metabolism and altered bone remodeling.

In a report of Hopwood et al. (Arthritis Research & Therapy (2007) 9: R100), microarray gene expression profiling of osteoarthritic bone suggests altered TGF-β/BMP signaling. GDF15 gene expression was downregulated in OA when compared with control bone.

A recent report of Iliopoulos et al (PLoS ONE (2008) 3:e3740) describes that GDF15 is a differential expressed protein between osteoarthritic and normal chondrocytes.

Repair of articular cartilage defects with suspensions of chondrocytes has been carried out in a variety of animal models and is now also employed in humans with degenerative joint disease. Autologous chondrocytes obtained from an unaffected area of the joint are released, expanded *in vitro* in the presence of autologous serum and subsequently injected in the cartilage defect. This procedure has led to a proven at least symptomatic amelioration. This conceptually promising approach has still wide margins for improvement, since it is known that *in vitro* expansion of chondrocytes results, after a limited number of cell divisions, in a loss of their phenotypic stability (as defined by the ability of chondrocytes to form hyaline *cartilage in vivo)* making the cell suspension to be injected unreliable. To date, however, it is not known how far it is possible to expand chondrocytes without hampering their phenotypic stability and therefore their capacity to form stable hyaline cartilage *in vivo,* resistant to vascular invasion and endochondral bone formation. Other factors that can affect the capacity of chondrocytes to form cartilage *in vivo* are the culture conditions, and several factors dependent on the donor such as age and pre-existing joint or systemic diseases. At the end of cell expansion the chondrocyte population is composed of some cells that retain their phenotypic stability, and others that still can proliferate but will not anymore contribute to cartilage repair. To obtain a consistent cell suspension for autologues chondrocyte transplantation (ACT), it is desirable to determine which is the actual capacity of the cells to form cartilage *in vivo* and, if necessary, to select stable chondrocytes within the expanded cell population. The importance of this issue is underscored by the large variability in the quality of the repair tissue obtained going from hyaline-like cartilage to fibrocartilage to no signs of repair.

Chondrocytes are the only normal skeletal cells known to grow anchorage-independent in agarose cultures (Benya and Shaffer. 1982, Cell 30:215-224). This culture system allows a recovery of some of the phenotypic traits that are lost with expansion in monolayer. Similar features as in agarose cultures, are observed in alginate bead cultures (Benz K et al., 2002, Biochem Biophys Res Comm 2002;293:284-92; Häuselmann HJ et al. 1994, Biochem Biophys Res Comm ,2002;293:284-92; Wang J et al, Osteoarthritis Cartilage. 2003;11:801-9). The expression of type 2 collagen and the capacity to grow and rescue phenotypic traits in agarose culture, are good assays to evaluate chondrocyte differentiation and the potential to differentiate respectively. However they do not measure the capacity of chondrocytes to form cartilage *in vivo.*

EP1498146 published on 19 January, 2005 provides an *in vivo* assay to measure the capacity of isolated chondrocytes to produce cartilage *in vivo* using a nude mouse model. This capacity is linked to a set of molecular markers associated with the outcome of joint surface defects ("JSD") repair in well-standardised animal models of JSD. The set of molecular markers (both membrane-associated and/or non membrane-associated) are also used as a final quality control for the cell suspension to be used for ACT or the repair of the cartilaginous structures. Results indicate the high expression of BMP-2, FGFR-3, and type II collagen as positively associated to chondrocyte stability, whereas activin-like kinase (ALK)-1 and collagen type X expression are negatively associated. During *in vitro* expansion of these chondrocytes, the molecular markers can only be determined by RT-PCR or immunohistochemistry and thus requires (the lysis) of precious cells.

There is a need to identify molecular markers associated with chondrocytes that would allow the clinician to produce suitable implants and to regenerate and repair cartilage tissue with the appropriate phenotypic stable cells and avoid scar formation to the greatest possible extent In an earlier application (PCT application WO2009/049913) the present applicant identified and characterized CRYAB and HSP27 as molecular markers in monitoring the chondrocyte stability of isolated or expanded cells. With the present invention, and in search of markers that can be determined in the cell culture media of the isolated or expanded cells, the applicant defines GDF15 as a secreted positive molecular marker for chondrocyte stability and as a tool to monitor, passage by passage, *in vitro* cell expansion and the manufacturing process of chondrocyte expansion. GDF15, optionally in combination with other markers for chondrocyte stability like CRYAB and HSP27, will be useful to optimize next generation chondrocyte expansion technologies, including expansion of chondrocyte-precursors, and chondrocyte derivatives, which express very low levels of or even no conventional chondrocyte differentiation markers such as BMP-2, COL2A1 and aggrecan. GDF15, again optionally in combination with other markers for chondrocyte stability like CRYAB and HSP27, will be useful to predict when cell expansion must be stopped, and especially to provide a quality control for chondrocytes to be used for ACT for lot release approval by the simple prelevation of cell culture medium, without the need to isolate precious cells.

### SUMMARY OF THE INVENTION

The present invention relates to a method for evaluating the phenotypic stability of isolated or expanded cells. Hence the present invention provides a method for evaluating the ability of isolated or expanded cells to produce, synthesize, organize or reorganize matrix components, said method comprising monitoring the expression of the molecular marker GDF15. In particular said method comprises monitoring a decrease; or a decrease and a subsequent increase in the expression of the molecular marker GDF-15. Said decrease can be down to about 10% to 30% of the initial GDF15 expression.

As used herein, the term "matrix components" refers to proteins synthesized by the chondrocyte that constitute the extracellular matrix of the cell such as aggrecan, collagens, proteoglycans and the like.

In an embodiment of the method of the invention, the isolated or expanded cells are pluripotent cells and/or chondrocytes. In particular said chondrocytes are derived from skeletal tissue, more in particular from hyaline cartilage or fibrocartilage.

Hence, in a further embodiment of the method of the invention, the matrix components are the typical constituents of the extracellular matrix of hyaline cartilage in particular said matrix components are COL2A1 and aggrecan.

In still a further embodiment of the method of the invention, said method further comprises monitoring of the expression of the molecular markers CRYAB and HSP27.

As further specified in PCT application WO2009/049913, a cell culture of isolated pluripotent cells and/or chondrocytes is identified as a phenotypic stable cell culture, by monitoring the transient decrease in CRYAB expression, and wherein up to the increase of CRYAB expression, said cell culture is identified as a phenotypic stable cell culture. In particular said transient decrease in CRYAB expression is at least 30% of the initial CRYAB expression; and in particular equals from about 40% to about 60% of the initial CRYAB expression in said cell culture of isolated pluripotent cells and/or chondrocytes.

As further specified in PCT application WO2009/049913, a cell culture of isolated pluripotent cells and/or chondrocytes is identified as a phenotypic stable cell culture, by monitoring HSP27 expression in said cell culture, and wherein an increase in HSP27 expression to a level of at least 140% of the initial HSP27 expression in said cell culture, is an indication of the dedifferentiation of said cell culture of isolated pluripotent cells and/or chondrocytes.

Thus in a particular embodiment of the present invention, a decrease in CRYAB expression down to about 70% or less of the initial CRYAB expression, an increase in HSP27 expression up to about 140% or more of the initial HSP27 expression, and a decrease down to about 20% of the initial GDF 15 expression, identifies the point up to which an isolated or expanded cell suspension is still capable to synthesize a functional cartilage matrix

In another embodiment of the method of the invention, the detection of expression of the molecular marker GDF 15, is performed with an immunoassay using antibodies, or fragments thereof, against GDF15. In particular said immunoassay is performed on cell culture supernates or cell culture lysates. Monitoring said expression of the molecular marker GDF15 can also be performed using a technique selected from the group of semi-quantitative RT-PCR, Northern hybridisation, differential display, subtractive hybridization, subtracted libraries, cDNA chips and cDNA arrays. Another aspect of the invention, relates to extending or enhancing the expression of matrix proteins of isolated or expanded cells, said method comprising adding GDF15 to said cells. In particular said matrix proteins are from hyaline cartilage, more in particular said matrix proteins are COL2A1 or aggrecan. Said isolated or expanded cells can be pluripotent cells and/or chondrocytes.

Another aspect of the invention relates to the use of the molecular marker GDF15 to predict the ability of isolated or expanded cells to synthesize matrix components and to determine when cell expansion must be stopped without loss of the ability of said cells to synthesize matrix components. The invention also provides the use of the molecular marker GDF15 in a cell quality control system or a quality control scoring algorithm. The described uses of the molecular marker GDF15 can be provided in combination with the expression of one, two or more of the molecular markers selected from the group consisting of COL2A1, BMP2 and aggrecan.

### BRIEF DESCRIPTION OF THE DRAWING

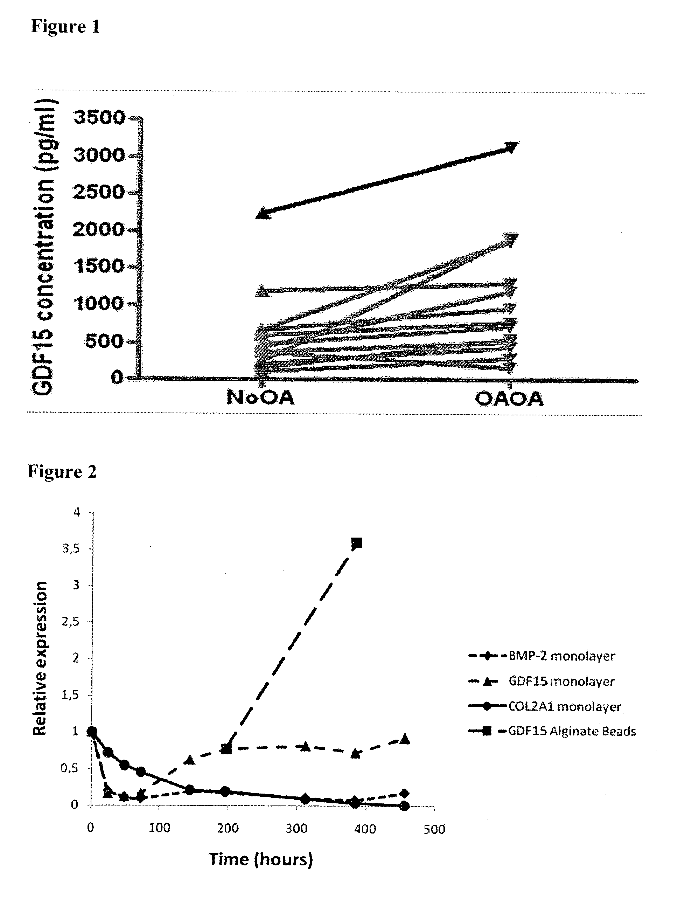

**Figure 1****:** Concentration of GDF15 in culture supernates from alginate cultured chondrocytes isolated from visually intact (NoOA) and visually damaged zones (OAOA) of OA-cartilage. A significant (p<0,01; Wilcoxon signed rank test) higher concentration was observed in the OAOA samples.
**Figure 2****:** Relative expression of Collagen type II (COL2A1), BMP-2 and GDF15 during monolayer expansion cultures. At time point 192h part of the cells were recultured in 3-D alginate beads, as described in the materials and methods section. Data points represent the mean relative expression of 3 different patients. Decreasing COL2A1 and BMP-2 levels indicate the time-dependent dedifferentiation of chondrocytes in monolayer culture. Encapsulation of expanded chondrocytes in a 3-D environment induces an increase in GDF 15 expression.

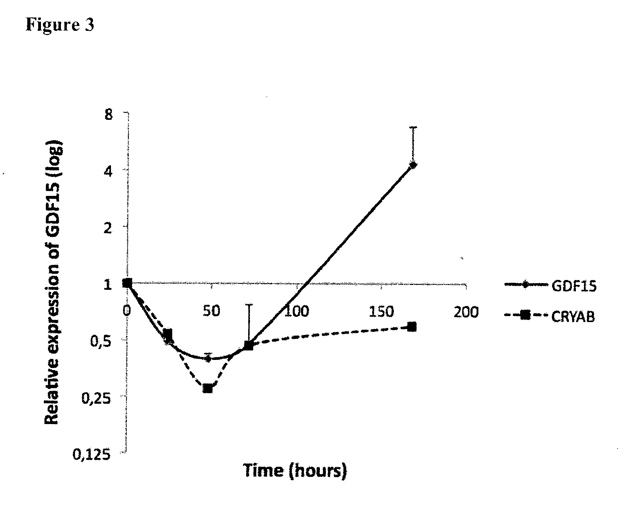

**Figure 3****:** Relative expression of GDF15 and CRYAB during monolayer expansion cultures. Meniscus chondrocytes were isolated and expanded as described. Expression levels of GDF15 and CRYAB were analyzed by QPCR as described. Data points represent the mean relative expression of 2 different patients.

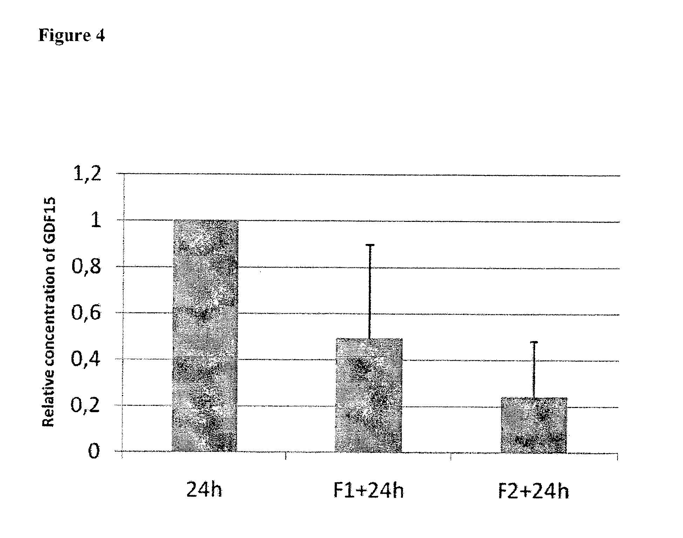

**Figure 4****:** GDF15 medium concentrations were determined by ELISA techniques at different time points: 24 hours after seeding of phenotypically stable chondrocytes, 24 hours after the first passage (F1 + 24 h) and 24 hours after the second passage (F2 + 24 h). Values are expressed as relative values to the first time point and represent the mean (± SD) relative concentration of 3 different patient samples. These data confirm the time dependent decreased protein secretion of GDF15 by expanding human articular chondrocytes.

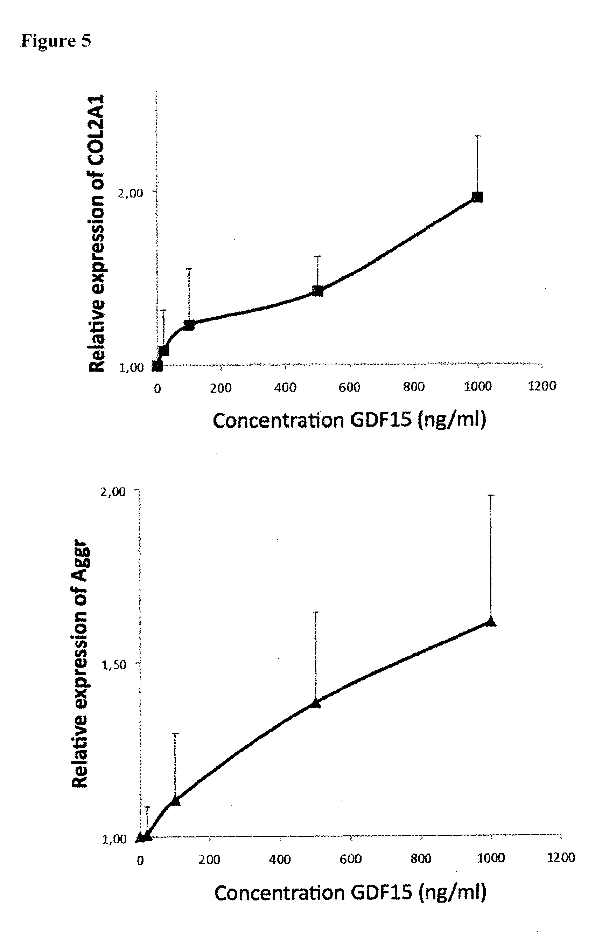

**Figure 5****:** Phenotypically stable human articular chondrocytes cultured in alginate beads were stimulated for 24 hours with hrGDF15 at different concentrations (ranging from 0 to 1000 ng/ml), demonstrating a concentration dependent increase in the expression of extracellular matrix genes upon GDF15 stimulation. Data points represent the mean (± SEM) relative expression of Collagen type II and aggrecan compared to unstimulated control cultures. Data represent the mean of 9 (except for 1000 ng/ml, n=6) independent experiments.

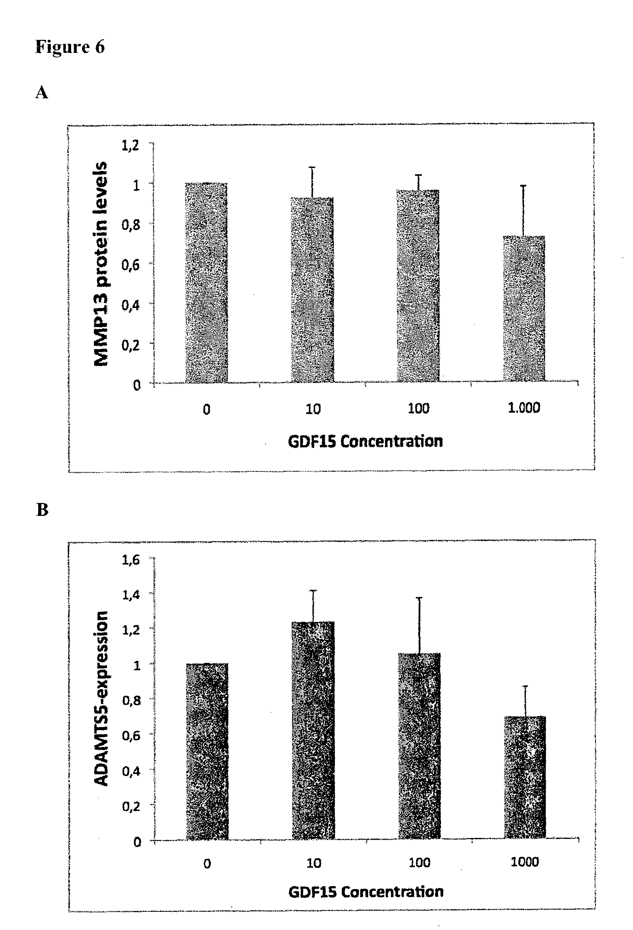

**Figure 6****:** A. Relative expression of MMP13, as determined by Western Blot, shows a decreasing trend upon stimulation of the cell cultures with increasing GDF15 concentrations. Data represent mean expression (±SEM) of 3 patient samples. B. Relative expression of ADAMTS-5 as determined by qPCR. Cell cultures stimulated with 1000 ng/ml show a reduced expression of ADAMTS-5, a major enzyme involved in aggrecan degradation. Data represent mean expression (±SEM) of 3 patient samples.

### DETAILED DESCRIPTION

With "chondrocyte phenotypic stability" is meant the capacity of a cell obtained from cartilage tissue or from any other tissue containing cells with chondrogenic potential to produce, synthesize, organize or reorganize a functional cartilage matrix, more in particular the capacity to synthesize matrix components of hyaline cartilage, even more in particular the capacity to synthesize at least the matrix components COL2A and/or aggrecan.

With "chondrogenic capacity" is meant the capacity to promote or stimulate the production of a functional cartilage matrix, more in particular to retain the expression of matrix proteins of hyaline cartilage, even more in particular to extend or enhance COL2A1 or aggrecan expression.

The term "functional cartilage matrix" refers to an extracellular matrix that provides enough elasticity and tensile strength as may be expected from hyaline cartilage. Typically, such matrix is rich in Collagen type II and proteoglycans.

With "molecular marker" is meant a polypeptide that distinguishes one cell (or set of cells) from another cell (or set of cells) in a population of cells and is associated to a peculiar biological function.

With "phenotypic stability" or "phenotypic stable" is meant the maintenance of the ability of any cell (e.g. a chondrocyte) to produce, synthesize, organize or reorganize, the components (e.g. matrix proteins) or the structure (e.g. matrix) of a specific tissue (e.g. cartilage).

With " stable cartilage" is meant cartilage not finally turning into bone, i.e. cartilage devoid of any signs of vascularization. Particularly, the stable cartilage in accordance with the present invention is human adult or mature articular cartilage but may also include animal adult or mature cartilage. Contrary to stable cartilage, transient cartilage in the end will become bone tissue. In the context of the present invention, cartilage is said to be stable if, even after e.g. seven weeks, any signs of bone formation are absent. Hence, hypertrophied chondrocytes do not form stable cartilage.

As used herein the "GDF15" polypeptide is meant to be a protein encoded by a mammalian *gdf15* gene, including allelic variants as well as biologically active fragments thereof containing conservative or non-conservative changes as well as artificial proteins that are substantially identical, i.e. 70%, 75%, 80%, 85%, 87%, 89%, 90%, 92%, 93%, 94%, 95%, 96%, 97%, 98%, or 99% identical to any one of the aforementioned GDF15 polypeptides. In a particular embodiment the GDF15 polypeptide is 70%, 75%, 80%, 85%, 87%, 89%, 90%, 92%, 93%, 94%, 95%, 96%, 97%, 98%, or 99% identical to the human GDF15 (encoded by Genbank Accession NM_004864(mRNA) or NC_000019.8 (genomic)).

By analogy, the "GDF15" polynucleotide is meant to include allelic variants as well as biologically active fragments thereof containing conservative or non-conservative changes as well as any nucleic acid molecule that is substantially identical, i.e. 70%, 75%, 80%, 85%, 87%, 89%, 90%, 92%, 93%, 94%, 95%, 96%, 97%, 98%, or 99% identical to any one of the aforementioned GDF15 encoding polynucleotides. In a particular embodiment the GDF15 polynucleotide is 70%, 75%, 80%, 85%, 87%, 89%, 90%, 92%, 93%, 94%, 95%, 96%, 97%, 98%, or 99% identical to the nucleic acid molecule encoding for human GDF15 (Genbank Acession N° NM_004864(mRNA) or NC_000019.8 (genomic)).

As used herein, the terms "polynucleotide" and "nucleic acid" are used interchangeably to refer polynucleotides of any length, either deoxyribonucleotides or ribonucleotides or analogs (e.g., inosine, 7-deazaguanosine, etc.) thereof. "Oligonucleotides" refer to polynucleotides of less than 100 nucleotides in length, preferably less than 50 nucleotides in length, and most preferably about 10-30 nucleotides in length. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can include modified nucleotides, such as methylated nucleotides and nucleotide analogs. If present, modifications to the nucleotide structure can be imparted before or after assembly of the polymer. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

"Polypeptide" refers to any peptide or protein comprising amino acids joined to each other by peptide bonds or modified peptide bonds. "Polypeptide" refers to both short chains, commonly referred to as peptides, oligopeptides or oligomers, and to longer chains, generally referred to as proteins. Polypeptides may contain amino acids other than the 20 gene-encoded amino acids.

"Polypeptides" include amino acid sequences modified either by natural processes, such as post-translational processing, or by chemical modification techniques which are well known in the art. Such modifications are well described in basic texts and in more detailed monographs, as well as in a voluminous research literature.

Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications (see, for instance, Proteins-Structure and Molecular Properties, 2nd Ed. , T. E. Creighton, W. H. Freeman and Company, New York, 1993; Wold, F. , Post- translational Protein Modifications: Perspectives and Prospects, pgs. 1-12 in Postranslational Covalent Modification of Proteins, B. C. Johnson, Ed. , Academic Press, New York, 1983; Seifter et al., "Analysis for protein modifications and nonprotein cofactors", Meth Enzymol (1990) 182: 626-646 and Rattan et al. , "Protein Synthesis: Post-translational Modifications and Aging", Ann NY Acad Sci (1992) 663: 4842).

### Method and use

This invention is based on the identification of GDF 15 as molecular actor in the homeostasis of chondrocytes, and accordingly provides GDF15 as molecular marker useful in monitoring the phentotypic stability of isolated pluripotent cells *in vitro.*

In a first objective, the present invention provides a method for evaluating the phenotypic stability of isolated or expanded cells, more in particular of isolated pluripotent cells and/or chondrocytes, said method comprising monitoring the expression levels of the molecular marker GDF 15 at regular time intervals. In a further embodiment the methods include determining the expression levels of GDF 15, COL2A1 and/or aggrecan at regular time intervals.

As used herein, the term "expanded cells" refers to the population of cells derived through proliferation of the isolated pluripotent cells and/or chondrocytes. "Pluripotent cells" are cells that can be induced to differentiate into all cell types except for extra embryonic tissue. In cell biology, the definition of pluripotency has come to refer to a cell that has the potential to differentiate into any of the three germ layers: endoderm (interior stomach lining, gastrointestinal tract, the lungs), mesoderm (muscle, bone, blood, urogenital), or ectoderm (epidermal tissues and nervous system). As used herein, it is further meant to include multipotent cells that have the potential to give rise to cells from multiple, but a limited number of lineages.

The "expression" generally refers to the process by which polynucleotides are transcribed into mRNA and/or the process by which the mRNA is subsequently translated into peptides, polypeptides or proteins. Hence the "expression" of a gene product, in the present invention of GDF15, COL2A1 and aggrecan can be determined either at the nucleic acid level or the protein level.

Detection can be by any appropriate method, including, e.g., detecting the quantity of mRNA transcribed from the gene or the quantity of nucleic acids derived from the mRNA transcripts. Examples of nucleic acids derived from an mRNA include a cDNA produced from the reverse transcription of the mRNA, an RNA transcribed from the cDNA, a DNA amplified from the cDNA, an RNA transcribed from the amplified cDNA, and the like. In order to detect the level of mRNA expression, the amount of the derived nucleic acid should be proportional to the amount of the mRNA transcript from which it is derived. The mRNA expression level of a gene can be detected by any method, including hybridization (e.g., nucleic acid arrays, Northern blot analysis, etc.) and/or amplification procedures according to methods widely known in the art. For example, the RNA in or from a sample can be detected directly or after amplification. Any suitable method of amplification may be used. In one embodiment, cDNA is reversed transcribed from RNA, and then optionally amplified, for example, by PCR. After amplification, the resulting DNA fragments can for example, be detected by agarose gel electrophoresis followed by visualization with ethidium bromide staining and ultraviolet illumination. A specific amplification of differentially expressed genes of interest can be verified by demonstrating that the amplified DNA fragment has the predicted size, exhibits the predicated restriction digestion pattern and/or hybridizes to the correct cloned DNA sequence.

In hybridization methods a probe, i.e. nucleic acid molecules having at least 10 nucleotides and exhibiting sequence complementarity or homology to the nucleic acid molecule to be determined, are used. It is known in the art that a "perfectly matched" probe is not needed for a specific hybridization. A probe useful for detecting mRNA is at least about 80%, 85%, 90%, 95%, 97% or 99% identical to the homologous region in the nucleic acid molecule to be determined. In one aspect, a probe is about 50 to about 75, nucleotides or, alternatively, about 50 to about 100 nucleotides in length. These probes can be designed from the sequence of full-length genes. In certain embodiments, it will be advantageous to employ nucleic acid sequences as described herein in combination with an appropriate label for detecting hybridization and/or complementary sequences. A wide variety of appropriate labels, markers and/or reporters are known in the art, including fluorescent, radioactive, enzymatic or other ligands, such as avidin/biotin, which are capable of giving a detectable signal. One can employ a fluorescent label or an enzyme tag, such as urease, alkaline phosphatase or peroxidase, instead of radioactive or other environmental undesirable reagents. In the case of enzyme tags, colorimetric indicator substrates are known that can be employed to provide a signal that is visible to the human eye or spectrophotometrically, to identify specific hybridization with complementary nucleic acid-containing samples.

Hence in an embodiment of the invention, monitoring the expression of the molecular marker GDF15 is performed using a technique selected from the group of semi-quantitative RT-PCR, Northern hybridisation, differential display, subtractive hybridization, subtracted libraries, cDNA chips and cDNA arrays.

Detection of the level of gene expression can also include detecting the quantity of the polypeptide or protein encoded by the gene. A variety of techniques are available in the art for protein analysis. They include but are not limited to radioimmunoassay (RIA), ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays and PAGE- SDS.

Antibodies that specifically recognize and bind to the protein products of these genes are required for these immunoassays. These may be purchased from commercial vendors or generated and screened using methods well known in the art. See e.g., Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al. eds, (1987)); the series METHODS IN ENZYMOLOGY (Academic Press, Inc.): PCR 2: A PRACTICAL APPROACH (MJ. MacPherson, B.D. Hames and G.R. Taylor eds. (1995)), Harlow and Lane, eds. (1988) ANTIBODIES, A LABORATORY MANUAL and ANIMAL CELL CULTURE (R.I. Freshney, ed. (1987)).

Hence, in an embodiment of the invention, monitoring the decrease or increase in the expression of the molecular marker GDF 15 is performed with an immunoassay using antibodies, or fragments thereof, against GDF15.

Anti-GDF15 antibodies and fragments thereof can be produced by any of the methods known to the art.

The antibodies can be bound to many different carriers and used to detect the presence of a GDF15 antigen. The antibodies can be suitable for use, for example, in immunoassays. The antibodies of the invention can be bound to many different carriers. The nature of the carrier can be either soluble or insoluble. Examples of well-knowm carriers include glass, polystyrene, polypropylene, polyethylene, dextran, nylon, amylases, natural and modified celluloses, polyacrylamides, agaroses and magnetic beads. Those skilled in the art will know of other suitable carriers for binding antibodies, or will be able to ascertain such, using routine experimentation.

In addition, the antibodies in these immunoassays can be detectably labeled in various ways. Examples of types of immunoassays, which can utilize antibodies, are competitive and noncompetitive immunoassays in either a direct or indirect format. Examples of such immunoassays are the radioimmunoassay (RIA) and the sandwich (immunometric) assay.

There are many different labels and methods of labeling known to those of ordinary skill in the art. Examples of the types of labels, which can be used in the present invention, include enzymes, radioisotopes, fluorescent compounds, colloidal metals, chemiluminescent compounds, phosphorescent compounds, and bioluminescent compounds. Those of ordinary skill in the art will know of other suitable labels for binding to an antibody, or will be able to ascertain such, using routine experimentation.

Another technique, which may also result in greater sensitivity, consists of coupling the antibodies to low molecular weight haptens. These haptens can then be specifically detected by means of a second reaction. For example, it is common to use such haptens as biotin, which reacts with avidin, or dinitrophenyl, puridoxal, and fluorescein, which can react with specific antihapten antibodies.

GDF15 is a secreted protein thus the immunoassays may be performed on cell culture supernates or cell culture lysates.

It has been found that dedifferentiation of isolated chondrocytes is accompanied with a decrease in GDF15 expression. This is particularly useful in monitoring the phenotypic stability of isolated or expanded chondrocytes, wherein the lowest GDF15 expression marks the start of a phenotypic change of said cells. As is shown in the examples hereinafter, the decrease of GDF15 expression in a dedifferentiating suspension of isolated or expanded chondrocytes is accompanied with a decreased expression of positive markers of a chondrogenic phenotype, such as BMP-2 and matrix genes including collagen type II (COL2A1) and aggrecan.

By "dedifferentiation" is meant the reduction of gene expression of genes expressed by a 'normal' articular chondrocyte (such as Collagen type II). "Redifferentation" is characterised by the normalization of the gene expression (both protein and nucleic acid level) of genes, normally expressed by an articular chondrocytes (such as Collagen type II).

Thus in one embodiment the present invention encompasses a method for evaluating the phenotypic stability of isolated or expanded cells, said method comprising monitoring the expression of the molecular marker GDF15.

In another embodiment of the present invention said method comprises monitoring a transient decrease of the expression of the molecular marker GDF15, wherein the lowest GDF15 expression marks the start of the phenotypic change of said isolated or expanded cells, i.e. it marks the dedifferentiation of said isolated or expanded cells, characterized in a reduced expression and ultimate loss in capability of said cells to synthesize a functional cartilage matrix, more in particular a reduced capacity to synthesize matrix components of hyaline cartilage, even more in particular a reduced capacity to synthesize at least the matrix components COL2A and/or aggrecan.

As is evident from the examples herein, and in an embodiment of the present invention, said transient decrease in GDF 15 expression can occur but is not limited to the first 24, 48, 72, 96 or 120 hours of isolation or expansion, and typically occurs within the first 48 hours of isolation. Said decrease without dedifferentiation can be down to about 20%, 30%, 40% or 50% of the initial GDF15 expression, in said isolated or expanded pluripotent cells and/or chondrocytes.

When the cell suspension of isolated pluripotent cells and/or chondrocytes cells is kept too long, the cells loose the capacity to synthesize a functional cartilage matrix, more in particular the capacity to synthesize matrix proteins of hyaline cartilage, even more in particular the capacity to synthesize at least COL2A and/or aggrecan.

Thus, in an even further embodiment, the method for evaluating the phenotypic stability of isolated or expanded cells and/or chondrocytes further comprise determining COL2A1, optionally in combination with aggrecan expression as molecular markers for chondrocyte phenotypic stability.

The monitoring of the expression of the molecular marker GDF15 in the methods of the invention can be combined with the monitoring of the expression of CRYAB and HSP27. In a particular embodiment, the point up to which an isolated or expanded cell suspension is identified as a phenotypic stable cell suspension, i.e. still capable to synthesize a functional cartilage matrix, can be characterized in a decrease in CRYAB expression down to about 70% or less of the initial CRYAB expression (the CRYAB expression measured at the start of the cell culture) and a decrease down to about 20% of the initial GDF15 expression; an increase in HSP27 expression up to about 140% or more of the initial HSP27 expression and a decrease down to about 20% of the initial GDF 15 expression; or a decrease in CRYAB expression down to about 70% or less of the initial CRYAB expression, an increase in HSP27 expression up to about 140% or more of the initial HSP27 expression, and a decrease down to about 20% of the initial GDF 15 expression.

Other embodiments of the invention are to the use of GDF15, either alone or in combination with other markers, to monitor cell expansion at different time points, namely to predict when cell expansion must be stopped and eventually to provide a means for quality control of chondrocytes to be used for cell transplantation ("ACT"), thus making chondrocyte suspensions for ACT a more reliable and consistent product.

Figure 2 clearly shows that GDF-15 expression varies during dedifferentiation of a chondrocyte cell culture. The end of a time frame, characterized by a transient decrease of GDF-15 expression, is indicative for the irreversible loss of the chondrocyte phenotype. As a consequence the measurement of GDF-15 expression levels at one given time point within said time frame (determined by any of the methods mentioned above) may be used in a quality control system for a(n) (expanded) chondrocyte culture. The expression level of GDF-15 at the specific time point can be between standardized intervals to pass quality control test. The standardized intervals will be specific for example but not limited to a culture condition, a laboratory or a procedure and can be determined by analyzing a group of samples under said specific culture conditions, of said specific laboratory or said specific procedure. The levels of GDF-15 expression can be used as such, relative to a reference gene/protein or related to a score. The latter can allow to combine GDF-15 expression levels with other gene expression levels in a general quality control scoring algorithm.

Hence the present invention provides for the use of the molecular marker GDF15 as a means for quality control of cells to be used for cell transplantation. Said use can be in combination with the expression of molecular marker COL2A1 and/or aggrecan.

Preferably, the outcome is linked to GDF15 expression levels. Preferably, the predictive value of GDF15 is further optimized, by analyzing the effect of independent variables (age, gender, background, co-morbidities). This can be done storing in a database all the data of the individual patient together with the expression of the molecular markers and a score that describes the outcome of the procedure (based on pain, function of the joint, stiffness of the repair tissue by indentometry, and eventually histologic and molecular analysis of biopsy of the repair tissue).

In order to determine a change in expression and to compare the expression of GDF15, COL2A1 and aggrecan the expression levels determined using any one of the aforementioned methods can be normalized vis-à-vis the expression of a household gene such as GAPDH, HPRT, PPIA, Actine and the ribosomal proteins L19 and L32, and compared to the expression at start of the cell culture, isolation or expansion of the cell suspension (t0). In a particular embodiment the expression levels are determined at least twice, more in particular at least 3, 4, 5, 6, 7, 8, 9, or 10 times over the period required to double the cell count in the cell culture.

As will be apparent to the skilled artisan, alternatively the expression levels of GDF15, COL2A1, BMP2 and aggrecan are compared with the 'control' expression level(s) of said genes in chondrogenic stable cells. These 'control' expression levels typically consist of the mean expression level(s) of said genes as determined in a representative set of isolated pluripotent cells and/or chondrocytes; preferably said 'control' levels are predetermined.

Thus, in a particular embodiment, the method comprises the step of comparing the expression levels of said genes with the predetermined (pre-established) control expression levels of said genes. The latter may be presented as a combined value on an incremental scale reflecting the chondrogenic capacity of isolated pluripotent cells and/or chondrocytes, using art known scoring models such as for described in WO2008061804: MARKER GENES FOR USE IN THE IDENTIFICATION OF CHONDROCYTE PHENOTYPIC STABILITY AND IN THE SCREENING OF FACTORS INFLUENCING CARTILAGE PRODUCTION. In said embodiment the method to monitor and assess the phenotypic stability of a cell expansion culture of isolated pluripotent cells and/or chondrocytes, comprises the step of determining the expression level(s) of GDF15 and/or COL2A1 and/or BMP2 and/or aggrecan in said cells and comparing said expression level(s) with the predetermined (pre-established) control expression level(s) of said gene(s).

Using the methods of the present invention, it is now possible to identify cells that retain their full chondrogenic phenotype, and that are suitable for the repair of connective tissue, including cartilage, in particular in the repair of cartilage degeneration associated with osteoarthritis.

The methods as provided herein, are particularly useful in monitoring the phenotypic stability of isolated chondrocytes, i.e. chondrocytes derived from skeletal tissue, such as for example derived from hyaline cartilage or fibrocartilage. The methods provided herein are particularly useful in monitoring the phenotypic stability of isolated pluripotent cells and/or chondrocytes including but not limited to pluripotent cells from mesenchymal tissue such as adipose tissue and muscle tissue, hyaline knee cartilage chondrocytes, synovial fibroblasts and meniscal chondrocytes.

As can be seen in figure 2, the initial decrease of GDF 15 expression is followed by an increase in GDF-15 expression. During cell expansion such an increase of GFD-15 expression after the transient decrease indicates the loss of the chondrocyte phenotype.

Hence, in an embodiment of the invention, GDF15 expression levels can be determined at different time points to monitor the expansion process under given experimental conditions (e.g. culture system, addition of growth factors, small molecules). Herein, the detection of an increase in GDF-15 expression, compared to a previous time point, is indicative for a loss of the chondrocyte phenotype. Said increase in GDF-15 expression after a decrease, can be but is not limited to, at least 10%, 20% or 30% of the GDF-15 expression measured at a previous timepoint. Monitoring said increase in GDF-15 expression after a transient decrease, may be particular useful in the optimization of for example but not limited to novel cell culture conditions or screening assays. Hereby eliminating the need to re-implant the cell culture in an animal model or 3-D system to evaluate the chondrogenic capacity of the cell culture.

Alternatively, said decrease without dedifferentiation can be down to about 5%, 10%, 15%, 20%, 30%, 40%, 50%, 60%, 70% or 80% of the initial GDF15 expression, in said isolated or expanded pluripotent cells and/or chondrocytes; and is typically down to about 10 to 30 % of the initial GDF 15 expression; more in particular down to about 15 to 25% of the initial GDF15 expression; even more in particular it can be down to about 20% of the initial GDF 15 expression. The pluripotent cells and/or chondrocytes thus characterized will still synthesize a functional cartilage matrix upon re-implantation in a 3-D environment.

Based on the above, it is also an object of the present invention to provide cell culture conditions or compounds capable of enhancing the phenotypic stability of a cell, said method comprising; applying the methods according to the invention in the presence and absence of the compound or culture condition to be tested, and determine whether said compound or cell culture condition is capable to prevent and/or delay the transient change in GDF-15 expression. In a particular embodiment the present invention provides a method to identify compounds or cell culture conditions capable of enhancing the chondrogenic capacity of a cell, said method comprising applying the methods according to the invention in the presence and absence of the compound or cell culture condition to be tested, and determine whether said compound or cell culture condition is capable to prevent and/or delay the transient decrease in GDF-15 expression, i.e. to extend the point up to which a cell expansion culture of said cells is identified as a phenotypic stable cell culture.

In a further embodiment of the present invention, the assays are used to identify specific markers co-expressed with GDF-15 and linked to the phenotypic stability of the cells.

### Cells and therapeutic application

In a particular embodiment the present invention provides a method for enhancing the phenotypic stability of isolated or expanded cells or a method for influencing the chondrogenic capacity of a cell. As described previously with "the chondrogenic capacity" of a cell is meant, the capacity to promote or stimulate the production of a functional cartilage matrix thus contributing to a stable cartilage tissue. Said method comprises adding GDF 15 to said suspension during isolation, expansion and/ or during orthopedic reconstructive surgery for the repair of connective tissue, in particular in the repair of cartilage, more in particular in the repair of cartilage degeneration associated with osteoarthritis. The capacity to promote or stimulate the production of a functional cartilage matrix is characterized in the capability to retain the expression of, matrix proteins such as but not limited to COL2A1 and aggrecan expression e.g. to extend or enhance COL2A1 and aggrecan expression. The cells can be pluripotent cells and/or chondrocytes.

Thus the present invention also provides a method for enhancing the phenotypic stability of isolated or expanded cells said method comprising adding GDF 15 to said cells, either directly or indirectly as part of the aforementioned orthopedic reconstructive surgery.

It is thus an object of the present invention to provide GDF15 for use in the repair of connective tissue, more in particular for use in the repair of cartilage, even more in particular for use in the repair of cartilage degeneration associated with osteoarthritis.

Thus one aspect of this embodiment includes a pharmaceutical composition comprising GDF15. For example, tissue-engineering protocols may include the application of bio-resorbable polymers (e.g. polylactic acid or polyglycolic avid) to fill the lesion. In such a use, a composition could contain a matrix coated or mixed with further growth factors, with in particular GDF15.

Methods for preparing the pharmaceutical composition according to the present invention can be found in "Remington's Pharmaceutical Sciences", 20th ed., Mid.Publishing Co., Easton, Pa., USA. (2000)

The agents, with in particular GDF 15 described herein can be packaged as a kit. Thus, one or more agents, with in particular GDF15 can be present in a first container, and the kit can optionally include one or more agents, with in particular GDF15, in a second container. The kit can include instructions describing the method of the present invention. The agents, cells, containers and/or the instructions can be present in a package.

The contents of the kit can contain but is not limited to GDF15, growth medium, buffers, multiwell plates, antibodies and/or enzyme substrates.

This invention will be better understood by reference to the Experimental Details that follow, but those skilled in the art will readily appreciate that these are only illustrative of the invention as described more fully in the claims. Additionally, throughout this application, various publications are cited.

### EXPERIMENTAL PART

### Example 1:

### Isolation and culture of human articular chondrocytes

Human articular chondrocytes were isolated as described by Verbruggen et al. [4]. OA affected cartilage was obtained from patients within 24 h from total knee arthroplasty. The cartilage from each of these patients was separated in visually intact cartilage (NoOA) and cartilage showing OA-lesions (OAOA). This study was approved by the local Ethics Committee. The cartilage obtained was diced into small fragments and chondrocytes were isolated by sequential enzymatic digestion (hyaluronidase, pronase, collagenase (Sigma-Aldrich, Steinheim, Germany)) [4]. Trypan blue exclusion revealed that >95% of the cells were viable after isolation.

Chondrocyte cultures in alginate beads were prepared as described by Guo *et al* [5], with some modifications [6]. The beads were maintained in a 6-well plate (20 beads/well; ± 50.000 chondroctyes/bead) containing DMEM (Gibco) with 10% fetal calf serum, antibiotics and antimycotics (Gibco) in an incubator at 37°C and in 5% CO₂. Medium was replaced three times a week for 10 days. After the culture period, the medium was aspirated and immediately frozen, and the alginate beads were washed and dissolved by incubation in 55 mM tri-sodium citrate dihydrate pH 6.8, at room temperature. The resulting suspension was centrifuged at 1500 rpm for 10 min to separate cells with their CAM from the constituents of the interterritorial matrix. The resulting cell-pellet was washed three times with PBS.

Concentration of GDF15 in culture supernates from alginate cultured chondrocytes isolated from visually intact (NoOA) and visually damaged zones (OAOA) of OA-cartilage were measured by standard ELISA techniques using the GDF15 ELISA DuoSet (R&D systems, Abingdon, UK) according to the manufacturer's instructions.

### Example 1 results:

### Elevated GDF15 concentrations in OA-patients and OA-chondrocte cultures

GDF15, secreted by human articular chondroctes isolated from visually intact (NoOA) and visually damaged (OAOA) zones of OA articular cartilage respectively, was analysed by measuring GDF15 concentrations in culture supernates of 13 different paired samples. Figure 1A shows a consistent higher concentration in chondrocyte cell cultures from visually damaged zones compared to visually intact zones (p<0,01; Wilcoxon signed rank test).

### Example 2:

### Dedifferentiation experiments

### Articular Chondrocytes

Phenotypically stable articular chondrocytes isolated from 3 OA-patients were seeded in monolayer culture at low density (20.000 cells/Cm²). When confluency was reached, cells were detached and reseeded at the initial density. At particular time points, cells were detached and encapsulated in alginate beads for 8 days, as described above. At indicated time points Trizol (Invitrogen) was added to the isolated cells, and RNA was extracted according to the manufacturer's instructions, followed by an additional purification step (RNeasy mini-kit (Qiagen)). This step included the digestion of DNA by deoxyribonuclease I (Invitrogen). cDNA was synthesized with oligo(dT) primers using the Superscript kit (Invitrogen).

### Meniscus Chondrocytes

Phenotypically stable meniscus chondrocytes, isolated from 2 OA-patients were seeded in monolayer culture at low density (20.000 cells/cm²) and allowed to expand for about 170 hours. At indicated time points Trizol (Invitrogen) was added to the isolated cells, and RNA was extracted according to the manufacturer's instructions, followed by an additional purification step (RNeasy mini-kit (Qiagen)). This step included the digestion of DNA by deoxyribonuclease I (Invitrogen). cDNA was synthesized with oligo(dT) primers using the Superscript kit (Invitrogen).

For both chondrocytes types, real-time PCR was performed using the ABI 7000 Sequence Detection System (Applied Biosystems). Each reaction utilized 5 µl of cDNA and a mixture of 20 µl of iTaq Supermix with Rox (Bio-Rad, Hercules, CA), TaqMan Gene expression assay (Applied Biosystems) and water. Each sample was performed in triplicate. The thermocycler conditions were 2 min at 50°C, followed by 2 min at 95°C and 45 cycles, each at 95°C for 15 s and 60°C for 1 min. Expression levels were normalized to those of human GAPDH, HPRT and PPIA. Relative quantization was calculated using the 2^{-ΔΔCt} method [7,8].

### Example 3:

### Western Blot analysis

Cell lysates of articular chondrocytes were prepared by resuspending cell pellets in 40mM Tris from the ReadyPrep Sequential Extraction Kit (Bio-Rad, Hercules, CA, USA), supplemented with 0,1 % SDS, containing protease inhibitors (Roche Diagnostics, Mannheim, Germany) and a phosphatase inhibitor-cocktail (Sigma-Aldrich, Steinheim, Germany). Cells were lysed by sonication and the proteins were isolated by centrifugation. Equal amounts (30 µg as determined by 2-D Quant kit, GE Healthcare, Fairfield, USA) were loaded on 10% SDS-PAGE gel. Equal loading was verified by Ponceau S staining (data not shown). MagicMark (Invitrogen, Paisley, UK) protein standards were run as molecular weight markers. Following 1-D gel electrophoresis, proteins were transferred to nitrocellulose membranes (Bio-Rad). The resulting membranes were immunostained with rabbit anti-GDF15 (Abcam, Cambridge, UK) followed by an anti-rabbit HRP-conjugated secondary antibody (Pierce, Rockford, IL, USA) and ECL chemiluminescence detection (Supersignal West Dura Extended Duration Substrate, Pierce). Chemiluminescence images were recorded using the VersaDoc-imaging system (Bio-Rad). Image analysis was performed by Quantity One v 4.4.0 (Bio-Rad).

### Example 2 and 3 results:

### GDF15 expression decrease during dedifferentiation of phenotypically stable chondrocytes

### Articular Chondrocytes

To evaluate the differentiation status dependent expression of GDF15 in articular chondrocytes, cells were allowed to dedifferentiate by seeding in a monolayer culture system. This system is generally known to induce dedifferentiation of articular chondrocytes, and this is clearly demonstrated by the time-dependent decreased expression of the differentiation markers Collagen type II (COL2A1) and BMP-2 (figure 2). As indicated in figure 2 seeding of phenotypically stable chondrocytes results in a quick and dramatic reduction in GDF15 mRNA expression, as determined by real-time RT-PCR. After 72 hours in monolayer culture, a limited increase in GDF15 expression is detected, which remains stable and which never reaches the initial GDF15 expression of phenotypically stable chondrocytes.

To imitate a 3-dimensional environment similar to re-implantation procedures associated with chondrocyte transplantation therapies, single passaged cell-cultures, which reached confluency were isolated and cultured in alginate beads. The 3-dimensional environment created by alginated beads, results in an increased expression of GDF15. Similar experiments were performed on additional patient samples, except that intracellular GDF15 protein levels were analyzed instead of mRNA transcript levels (data not shown). On the protein level, only phenotypically stable chondrocytes show an indisputable positive signal. As might be expected from the mRNA data, culture of expanded chondrocytes in a 3-D environment results in an increase in GDF 15 protein levels (data not shown). In addition, figure 4 indicates that the expression of GDF15 may be analysed by the measurement of secreted protein concentrations. This opens up perspectives to follow culture quality by the simple prelevation of cell culture medium, without the need to isolate precious cells.

### Meniscus Chondrocytes

In a comparable experiment, the differentiation status dependent expression of GDF15 was assessed in meniscus chondrocytes. Thereto cells were allowed to dedifferentiate by seeding in a monolayer culture system. As indicated in figure 3 seeding of phenotypically stable chondrocytes results in a quick and dramatic reduction in GDF 15 and CRYAB mRNA (complementary marker) expression, as determined by real-time RT-PCR. A minimal levels of expression of both genes was observed at 48 hours.

### Example 4:

### GDF15 stimulation experiments

Chondrocytes were cultured in alginate beads as described above. After a 10-day culture period, chondrocyte cultures were stimulated with different concentrations of human recombinant GDF 15 (R&D systems, Abingdon, UK; Peprotech, London, UK) or vehicle only for 24 hours. Thereafter, culture medium was aspirated and frozen at - 80°C until further use. Cells were isolated and mRNA was extracted as described above. Gene expression was analysed by real-time RT-PCR as described earlier.

### Example 4 results:

### Erogenous human recombinant GDF15 promotes the expression of extracellular matrix genes

At this time, the molecular function of GDF-15 in articular chondrocytes is unknown. To provide deeper insights herein, different concentrations of GDF15 were added to alginate cell cultures (ranging from 0 to 1000 ng/ml) from 9 different patients who underwent a total-knee prosthesis (except concentration 1000 ng/ml, n=5). On average, a concentration dependent increase in Collagen type II and aggrecan expression was observed. As both are important constituents of the extracellular matrix (ECM), the functional unit of articular cartilage, these experiments indicate that GDF15 may serve as an anabolic mediator in articular chondrocytes that may promote ECM synthesis. Thus, indicating that GDF15 may be used as additive in expansion/redifferentiation cultures, to obtain a higher quality chondrocyte cell culture, i.e. expressing higher levels of Col2A1 and aggrecan.

In addition the effect of GDF15 stimulation on the expression of MMP13 and ADAMTS5 was assessed in chondrocyte cultures of 3 patients. The expression of these major enzymes involved in cartilage degeneration reduces upon increasing GDF concentration (Figure 6).

Finally, GDF15 stimulated chondrocyte cultures from 2 different patient samples were analyzed for aggrecan catabolism by determining the expression of the 21kDa and 58kDa aggrecan fragments. Expression of both fragments decreased upon increasing GDF 15 concentrations (data not shown).

In conclusion, these data indicate that GDF 15 stimulation not only induces the expression of matrix genes (COL2A1 and Aggr), but can also prevent its catabolism (Aggr). Furthermore, it contributes to the reduction of expression of genes involved in cartilage degeneration (MMP13 and ADAMTS5). In other words, the examples show an anabolic and anti-catabolic activity of GDF 15 in vitro. These experiments were conducted on human articular chondrocytes seeded in a 3-D alginate beads. It is generally accepted that the phenotype of cells cultured in these beads is representative for the in vivo phenotype. [9]

Animal experiments will be conducted to confirm the therapeutic activity of GDF15 in vivo. GDF15 -/- mice (Characterization of Growth-Differentiation Factor 15, a Transforming Growth Factor b Superfamily Member Induced following Liver Injury, EDWARD C. HSIAO, LEONIDAS G. KONIARIS, TERESA ZIMMERS-KONIARIS, SUZANNE M. SEBALD, THANH V. HUYNH, AND SE-JIN LEE May 2000, p. 3742-3751 Vol. 20, No. 10) will be compared to heterozygous (GDF15 +/-) and wild-type littermates (GDF15 +/+). Several clinical, biochemical and histological parameters related to the development of cartilage degeneration will be analyzed at the age of 3, 6 and 12 months. Heterozygous mice serve as a control to check whether partial restoration of GDF15 expression slows down or inhibits cartilage degeneration.

### References

[1] Arnett, F. C., Edworthy, S. M., Bloch, D. A., McShane, D. J., et al., Arthritis Rheum. 1988, 31, 315-24.
[2] Dougados, M., van-der-Linden, S., Juhlin, R., Huitfeldt, B., et al., Arthritis Rheum. 1991, 34, 218-27.
[3] Altman, R., Asch, E., Bloch, D., Bole, G., et al., Arthritis Rheum. 1986, 29, 1039-49.
[4] Verbruggen G, Wang J, Wang L, Elewaut D, Veys EM. Analysis of chondrocyte functional markers and pericellular matrix components by flow cytometry. Totowa, New Jersey: Humana Press; 2004.
[5] Guo JF, Jourdian GW, MacCallum DK. Culture and growth characteristics of chondrocytes encapsulated in alginate beads. Connect Tissue Res. 1989;19(2-4):277-97.
[6] Verbruggen G, Veys EM, Wieme N, Malfait AM, Gijselbrecht L, Nimmegeers J, et al. The synthesis and immobilisation of cartilage-specific proteoglycan by human chondrocytes in different concentrations of agarose. Clin Exp Rheumatol. 1990;8(4):371-8.
[7] Livak KJ, Schmittgen TD. Analysis of relative gene expression data using real-time quantitative PCR and the 2(-Delta Delta C(T)) Method. Methods 2001;25(4):402-8.
[8] Vandesompele J, De Preter K, Pattyn F, Poppe B, Van Roy N, De Paepe A, et al. Accurate normalization of real-time quantitative RT-PCR data by geometric averaging of multiple internal control genes. Genome Biol 2002;3(7):34.
[9] Hafselmann et al., Am. J. Physiol., 1996; 271: C742-752.

## Claims

1. An in vitro method for evaluating the ability of isolated or expanded cells to produce, synthesize, organize or reorganize matrix components, said method comprising monitoring the expression of the molecular marker growth differentiation factor 15 (GDF15).

2. The method as claimed in claim 1 said method comprising monitoring a decrease in view of the initial GDF15 expression, or a decrease and a subsequent increase in view of the initial expression of the molecular marker GDF15.

3. The method as claimed in any one of claims 1 to 2 wherein the cells are pluripotent cells and/or chondrocytes.

4. The method as claimed in any one of claims 1 to 3 wherein the matrix components are the typical constituents of the extracellular matrix of hyaline cartilage.

5. The method as claimed in any one of claims 1 to 4, further comprising monitoring the expression of the molecular markers CRYAB and HSP27.

6. A method to extend or enhance the expression of matrix proteins or to suppress or slow down degradation of matrix proteins of isolated or expanded cells, said method comprising adding GDF15 to said cells.

7. The method of claim 6 wherein said matrix proteins are the typical constituents of the extracellular matrix of hyaline cartilage.

8. The method of claim 6 or 7 wherein the cells are pluripotent cells and/or chondrocytes.

9. Use of the molecular marker GDF 15 to predict the ability of isolated or expanded cells to synthesize matrix components and to determine when cell expansion must be stopped without loss of the ability of said cells to synthesize matrix components.

10. Use of the molecular marker GDF15 as claimed in claim 9 in a cell quality control system or a quality control scoring algorithm.

11. GDF15 for medical use in the repair of connective tissue, in particular in the repair of cartilage, more in particular in the repair of cartilage degeneration associated with osteoarthritis.

## Patentansprüche

1. Ein in-vitro-Verfahren zur Bewertung der Fähigkeit isolierter oder erweiterter Zellen zur Herstellung, Synthetisierung, Organisation oder Reorganisation von Matrixkomponenten, wobei das besagte Verfahren eine Beobachtung der Expression des molekularen Markers Wachstums- und Differenzierungsfaktor 15 (GDF15) umfasst.

2. Das Verfahren nach Anspruch 1, wobei das besagte Verfahren eine Beobachtung der Abnahme im Hinblick auf die ursprüngliche Expression von GDF15 oder der auf eine Abnahme folgenden Zunahme im Hinblick auf die ursprüngliche Expression des molekularen Markers GDF15 umfasst.

3. Das Verfahren nach einem der Ansprüche 1 oder 2, wobei es sich bei den Zellen um pluripotente Zellen und/oder Chondrozyten handelt.

4. Das Verfahren nach einem der Ansprüche 1 bis 3, wobei es sich bei den Matrixkomponenten um die typischen Bestandteile der extrazellulären Matrix von hyalinem Knorpel handelt.

5. Das Verfahren nach einem der Ansprüche 1 bis 4, das ferner die Beobachtung der molekularen Marker CRYAB und HSP27 umfasst.

6. Ein Verfahren zur Erweiterung oder Anreicherung von Matrixproteinen oder zur Unterdrückung bzw. zur Verzögerung des Abbaus der Matrixproteine isolierter oder erweiterter Zellen, wobei das besagte Verfahren die Zugabe von GDF15 zu den besagten Zellen umfasst.

7. Das Verfahren nach Anspruch 6, wobei es sich bei den besagten Matrixproteinen um die typischen Bestandteile der extrazellulären Matrix von hyalinem Knorpel handelt.

8. Das Verfahren nach einem der Ansprüche 6 oder 7, wobei es sich bei den Zellen um pluripotente Zellen und/oder Chondrozyten handelt.

9. Die Verwendung des molekularen Markers GDF15 zur Prognose der Fähigkeit isolierter oder erweiterter Zellen zur Synthetisierung von Matrixkomponenten und zur Ermittlung des Zeitpunktes, an welchem die Zellerweiterung anzuhalten ist, sollen die besagten Zellen nicht ihre Fähigkeit zur Synthetisierung von Matrixkomponenten einbüßen.

10. Die Verwendung des molekularen Markers GDF15 gemäß Anspruch 9 in einem System zur Kontrolle der Zellqualität oder einem der Qualitätskontrolle dienenden Scoring-Algorithmus.

11. Die medizinische Verwendung von GDF15 zum Zwecke der Reparatur von Bindegewebe, insbesondere der Reparatur von Knorpeln, und noch spezifischer der Reparatur einer mit Osteoarthritis in Verbindung zu bringenden Degeneration des Knorpels.

## Revendications

1. Une méthode in vitro pour évaluer la capacité de cellules isolées ou expansées à produire, synthétiser, organiser ou réorganiser les composants de la matrice, ladite méthode comprenant le contrôle de l'expression du facteur 15 (GDF15).

2. La méthode selon la revendication 1 et dite méthode contrôlant une réduction en vue des l'expression initiale GDF15, ou une réduction et une augmentation subséquente en vue de l'expression initiale du marqueur moléculaire GDF15.

3. La méthode selon la revendication de n'importe laquelle de revendications 1 à 2 où les cellules sont pluripotentes et/ou chondrocytes.

4. La méthode selon la revendication de n'importe laquelle des revendications 1 à 3 où les composants de la matrice sont les constituants habituels de la matrice extra cellulaire de cartilage hyalin.

5. La méthode selon les réclamations 1 à 4, comprenant en plus un contrôle de l'expression des marqueurs moléculaires CRYAB et HSP27.

6. Une méthode pour étendre ou pour renforcer l'expression des protéines matricielles ou pour supprimer ou ralentir la dégradation des protéines matricielles des cellules isolées ou expansées, ladite méthode comprenant un ajout de GDF15 aux dites cellules.

7. La méthode de la revendication 6 où lesdites protéines matricielles sont les constituants typiques de la matrice extracellulaire de cartilage hyalin.

8. La méthode des revendications 6 ou 7 où les cellules sont des cellules pluripotentes et/ou des chondrocytes.

9. L'utilisation du marqueur moléculaire GDF15 pour prévoir la capacité des cellules isolées ou expansées de synthétiser les composants de la matrice et de déterminer quand il faut arrêter l'expansion de la cellule sans perdre la capacité desdites cellules pour synthétiser les composants de la matrice.

10. L'utilisation du marqueur moléculaire GDF15 selon la revendication 9 dans un système de contrôle de la qualité de la cellule ou un algorithme de mesure de contrôle de qualité.

11. GDF15 pour une utilisation moléculaire dans la réparation du tissue conjonctif, en particulier dans la réparation du cartilage, en particulier dans la réparation du cartilage de dégénération associé à l'arthrose.
